# EUROPEAN PATENT SPECIFICATION

(11) **EP 4 249 047 B1**
(45) Date of publication and mention of the grant of the patent: **20.05.2026**
(21) Application number: 23162618.5
(22) Date of filing: 17.03.2023
(51) Int. Cl.: A61N 2/00

(54) **DELIVERY OF THERAPEUTIC PAIRED PULSE TRANSCRANIAL MAGNETIC STIMULATION**
ABGABE EINER THERAPEUTISCHEN TRANSKRANIELLEN MAGNETISCHEN STIMULATION MIT GEPAARTEN IMPULSEN
ADMINISTRATION DE STIMULATION MAGNÉTIQUE TRANSCRÂNIENNE PAR IMPULSIONS APPARIÉES THÉRAPEUTIQUES

(30) Priority: 21.03.2022 FI 20225247
(43) Date of publication of application: 27.09.2023
(73) Proprietor: Nexstim Oyj, 00510 Helsinki (FI)
(72) Inventor: JÄRNEFELT, Gustaf, 00510 Helsinki (FI)
(74) Representative: Laine IP Oy

(56) References cited:
- WO-A1-2014/130071
- US-A1- 2008 200 749
- US-A1- 2015 018 692
- US-A1- 2019 201 707
- US-A1- 2019 217 116
- JUNG N ET AL: "FV 5 Quadri-pulse theta burst stimulation using ultra-high frequency bursts at I-wave periodicity induces direction dependent bi-directional plasticity in human primary motor cortex", CLINICAL NEUROPHYSIOLOGY, vol. 128, no. 10, 31 December 2017 (2017-12-31), XP085189035, ISSN: 1388-2457, DOI: 10.1016/J.CLINPH.2017.06.047

## Description

### FIELD

The present disclosure relates to the field of transcranial magnetic stimulation, TMS.

### BACKGROUND

Transcranial magnetic stimulation entails delivery of electromagnetic fields through a cranium into the intracranial space, where the delivered electromagnetic field will interact with tissue, in particular, the delivered electromagnetic field may interact with brain tissue.

The electromagnetic field may be delivered in pulsed form, such that the field is not a constant field. The maximum amplitude of the pulse may be selected based on the aim of the TMS session to be delivered. Likewise, the pulses may be delivered in pairs and the temporal duration of the pulses may be selected to enhance the results that are sought from the TMS session.

Document US2008/0200749 A1 discloses a TMS pulse train which repeats 20 pulses at a pulse interval of 1,792 ms, with a pause of 161 ms between such pulse trains. Jung N. et al.: "Quadri-pulse theta burst stimulation using ultra-high frequency bursts at I-wave periodicity induces direction dependent bi-directional plasticity in human primary motor cortex", CLINICAL NEUROPHYSIOLOGY, vol. 120, no. 10 discloses a TMS pulse train comprising quadruplets.

### SUMMARY OF THE INVENTION

The invention is defined by the features of the independent claim. Some specific embodiments are defined in the dependent claims.

### SUMMARY OF THE DISCLOSURE

According to a first aspect of the present disclosure, there is provided an apparatus for the delivery of a session of therapeutic transcranial magnetic stimulation, TMS, the apparatus comprising at least one processing core, at least one memory including computer program code, the at least one memory and the computer program code being configured to, with the at least one processing core, cause the apparatus at least to: define a session of transcranial magnetic stimulation, TMS, the session of TMS comprising a plurality of pulse trains, each pulse train comprising at least one burst and each burst comprising at least one pulse pair, and send signals configured to cause delivery of the session of TMS, wherein each pulse train comprises 5 to 20 bursts, the interval between bursts being 80 to 500 ms, the interval between pulses of the at least one pulse pair is 1 to 5 ms, and the interval between pulse trains is 1 to 60 seconds.

According to a second aspect of the present disclosure, there is provided an apparatus comprising: means for defining a session of transcranial magnetic stimulation, TMS, the session of TMS comprising a plurality of pulse trains, each pulse train comprising at least one burst and each burst comprising at least one pulse pair; means for placing a TMS device relative to an individual's brain such that a focus of the TMS device targets a selected region within the brain; and means for applying the session of TMS using the TMS device, wherein each pulse train comprises 5 to 20 bursts, the interval between bursts being 80 to 500 ms, the interval between pulses of the at least one pulse pair is 1 to 5 ms, and the interval between pulse trains is 1 to 60 seconds.

According to a third or fourth aspect of the present disclosure, there is provided a non-transitory computer readable medium having stored thereon a set of computer readable instructions that, when executed by at least one processor, cause an apparatus to at least: cause a method in accordance with at least one embodiment of the present disclosure to be performed or a computer program configured to cause a method in accordance with at least at least one embodiment of the present disclosure to be performed.

### BRIEF DESCRIPTION OF THE DRAWINGS

FIGURE 1 illustrates an example system in accordance with at least some embodiments of the present invention;
FIGURE 2A illustrates an example pulse sequence in accordance with at least some embodiments of the present invention;
FIGURE 2B illustrates an example pulse sequence in accordance with at least some embodiments of the present invention;
FIGURE 3 illustrates an example apparatus capable of supporting at least some embodiments of the present invention, and
FIGURE 4 is a flow chart illustrating a sequence of events in accordance with at least some embodiments of the present invention.

### EMBODIMENTS

In transcranial magnetic stimulation, TMS, electromagnetic pulses of stimulation are applied to a brain through a skull of the person receiving the session of TMS. The effects of TMS can be enhanced, it has been discovered, by arranging pulses in sequences. In detail, pulses may be provided singly as isolated pulses or as a set of pulses such as paired pulses, triplets or quadruplets. Further, the pairs themselves may be arranged into bursts, and bursts in turn may be provided as pulse trains. A set of one or more, for example two or more pulse trains forms a session of TMS.

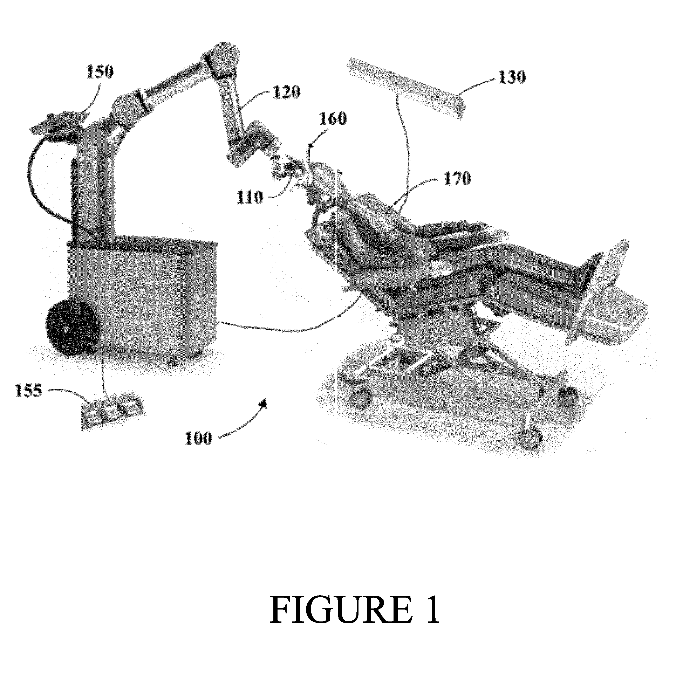

FIGURE 1 illustrates an example system in accordance with at least some embodiments of the present disclosure. In detail, the system illustrated in FIGURE 1 is of a type which may be used to deliver a session comprising the pulse trains which will be disclosed here. FIGURE 1 illustrates an apparatus 100 for facilitating delivery of TMS. As shown, the apparatus comprises: a controller 150 configured to be functionally connected to a tracking system 130 and a multi-axis robotic arm 120 having a TMS stimulation device 110 affixed thereto. The controller 150 may be configured to use anatomical data concerning person 170, said anatomical data defining a shape of the cranium and/or the brain of the person. The controller may be configured to receive information from the tracking system 130 regarding a real-time position and orientation of the head of person 170, and control the multi-axis robotic arm 120 to maintain the TMS stimulation device 110 at an optimal position and orientation relative to the head based on the anatomical data and the information regarding the real-time position and orientation of the head, in order to target a selected region within the brain for stimulation with the TMS stimulation device 110. The selected region may be near the surface of the brain, for example. As seen in FIGURE 1, the optimal position and orientation is such that a surface of the TMS stimulation device 110 is a predetermined distance 160 from the outer surface of the skin.

The anatomical data, where used, may be derived from medical imaging, such as at least one of: magnetic resonance imaging MRI, computed tomography CT, X-Ray, and ultrasound. Such medical imagery may carry information which can be directly interpreted as representing the cranium and skin of an individual. In other embodiments, the anatomical data may simply reflect the outer shape of the head of person 170. Additionally or alternatively, the anatomical data may carry information on brain structure inside the cranium, which may be needed for positioning of TMS stimulation device 110 depending on the specific aim of the TMS session.

A wide variety of tracking systems are available for use with embodiments of the present disclosure. For example, a tracking system according to certain embodiments of the present disclosure is configured to track markers applied to the head of the person 170 using an optical scanner, such as a video camera or stereo video camera. Such markers may be applied in a consistent location based on anatomical landmarks, for example, in order to provide a consistent mapping of anatomical data to the real-time position and orientation of the head of person 170.

At least some embodiments of the present disclosure employ image recognition in order to track at least one of a subject's head and TMS stimulation device 110. For example, a camera may be employed to capture images of the TMS stimulation device and/or subject's head and then image recognition techniques can derive a current position and orientation for the TMS stimulation device and/or subject's head. Certain embodiments employ artificial intelligence, AI, or machine learning techniques to better track objects via image recognition. Some embodiments use image recognition in order to co-register the real-time information regarding the position and orientation of the patient's head with the anatomical data.

A foot pedal 155 as illustrated in FIGURE 1, or another kind of user interface input device, may be used by an operator to configure apparatus 100 to a state when TMS pulses may be administered, and to another state when the generation of TMS pulses is not possible. In particular, a session of TMS may take place after an operator provides an input using the user interface device, such as foot pedal 155.

It has been surprisingly discovered, that, more than the number of pulses, the inter-pulse interval or sequence of intervals between pulses provides a large impact in TMS session effectiveness and/or impact. For example, a marked increase in impact has been discovered when pulse pairs are used in place of single pulses. The inter-pulse interval between the two pulses in a pulse pair may be selected from the range between 1 to 20 milliseconds, for example. This increased impact of pulse pairs provides a variety of benefits. For example, it allows for the use of lower intensity stimulation which provides the same result as higher-intensity stimulation with a different inter-pulse interval. This lowered intensity results in less unwanted effects on the surface musculature and potentially the eyes, depending on stimulation target. This increases patient comfort while achieving the same result.

The interval between two pulses of a pulse pair, or inter-stimulus interval, the ISI, may be 1 to 5 milliseconds, ms, such as 1 to 2,8 ms, such as 1,2 to 1,7 ms, for example 1,4 or 1,5 ms. The inter-pair interval between two pulse pairs in a burst may be between 10 and 30 ms, for example, between 13 and 25 ms, such as between 19 and 21 ms. The inter-burst interval between two bursts in a pulse train may be between 80 and 500 ms, for example between 100 and 300 ms, such as from 250 to 320 ms. In particular, the inter-burst interval may be from 140 to 200 ms or from 155 to 165 ms. The inter-train interval between two pulse trains in a session may be between 4 and 30 seconds, for example between 6 and 20 seconds, such as between 7 and 9 seconds, such as 8 seconds, for example. The interval between pulse pairs may vary within the session, for example, within an individual burst or between bursts. Also the inter-train interval may vary within the session. Ranges discloses in this paragraphs may be combined with each other and all their combinations are hereby expressly disclosed. The interval between pulse pairs may be measured from the first pulse of one pulse pair to the first pulse of the next pulse pair.

As an example of variation in ISI, there may be three pulse pairs within one burst, wherein the first pulse pair having an interval between pulses of 1.4 ms, the second pulse pair an interval of 1.7 ms and the third pulse pair an interval of 1.2 ms. As a further example, a first burst may comprise pulse pairs all having an interval between pulses of 1.4 ms, while a second burst has pulse pairs with an interval between pulses of 1.7 ms and a third burst has an ISI interval of 1.2 ms. The variation of interval between pulses of the pulse pair, or interpulse interval, may be used to determine an optimal interpulse interval for an individual. For example for an individual on certain day. As another example, involving a pre-treatment step of applying pulse pairs having varied interpulse intervals and measuring the response to each such pulse pair to determine an optimal interpulse interval. Said optimal interpulse interval can then be used to define the session of TMS. Variation of the interval between pulses of the pulse pair may provide for utilization of the optimal interval of at least some pulse pairs during a session. For example, the optimal interval may change throughout a session and variation ensures that at least some pulse pairs have an optimal interval throughout a session. Further, variation of the interval may facilitate phase difference of stimulation to brain activity frequency. As a further example, a session may comprise pulse trains with different ISI.

As an example of varying inter-train interval, the second train is 4 seconds after the first, the third is 12 seconds after the second, the fourth 20 seconds after the third.

The number of pulse pairs in a burst may be two or three, for example. The number of bursts in a pulse train may be from 2 to 20, for example from 4 to 18, such as 9 to 12. The number of bursts per train need not be the same in each train in a session, rather, the number of bursts per train may decrease or increase as the session progresses. In particular, a pulse train may comprise ten or twenty bursts of three pulse pairs each with 160ms between the bursts. Alternatively, a train may comprise ten or twenty bursts of two pulse pairs each, with 180ms between bursts.

The number of pulse trains per session may be eight or at least eight, such as from 10 to 40, for example. The session may last between one and eight minutes, or between three and 15 minutes when the session comprises 20 pulse trains, for example.

Within certain TMS sessions wherein 3 pulse pairs are used per burst, the interval between pulse pairs is between 20 and 30 ms, while in other sessions, the interval between pulse pairs may be as low as 10 ms. Certain TMS sessions have an interval between pulse pairs set by the limitations of the TMS system being employed. For example, if a TMS system does not have sufficient energy storage or power electronic capability, the interval between pulse pairs may be increased until the pulse pairs are delivered consistently. In certain methods, the interval between pulse pairs is selected such that, while the TMS system cannot deliver consistent pulse pairs, the variation or roll off, is within predetermined limits.

The expression "pulse pair" is adopted as a terminological convention in the present disclosure. In general, pulse pairs may comprise pulse triplets or pulse quadruplets, technically sets of individual pulses. In a triplet or quadruplet, the same inter-pulse intervals, ISI, may be used as in pairs with two and only two pulses.

The amplitude of pulses in a pulse pair may be equal, or un-equal. As an example of un-equality, the second pulse in a two-pulse pair may be between 50% and 180% of the amplitude of the first pulse in the pair. Amplitudes of pulses in pulse pairs with three or four pulses may also be unequal. For example, in a three-pulse pair or four-pulse pair, each pulse after the first one may have a greater amplitude than the immediately preceding one.

A session may comprise biphasic or monophasic pulses. In some embodiments, a session comprises both biphasic and monophasic pulses. Methods may employ a variety of pulses, such as, for example, biphasic or monophasic pulses. In at least some embodiments, both biphasic and monophasic pulses are employed during a session of TMS.

In some embodiments, session timings described above are selected based on brain activity and/or frequencies measured by electroencephalography, EEG, or other ways. For example, EEG may be used to determine that a pulse should be delivered immediately, or that the defined session, comprising plural pulse trains, should be started immediately. As a further possibility, an inter-train interval may be dynamically adapted during the inter-train interval in question, based on brain activity measurement. For example, it is thus possible to vary a start time of a pulse train by synchronizing to brain activity. Delaying or advancing the start of the next pulse train may be done with a selected time adjustment to synchronize with brain activity by up to +-3 seconds relative to the nominal inter-train interval, such that a nominally eight-second inter-train interval may be rendered as a five- or eleven-second interval, for example. The start of the train may be synchronized with brain activity, that is, the start of a train can be delayed or advanced in order to synchronize with brain activity. Sometimes this is done within a time limit, for example, within 3 seconds of a predetermined start time. For example, if the predetermined interval between trains was 8 seconds, the method would synchronize to brain activity so long as the train started between 5 and 11 seconds after the previous train. As another example, if the first train is desired to synchronize with brain activity, the first train and thus the session may be started whenever brain activity is determined to be suitable. For example, the timing synchronization may be achieved by monitoring for a predetermined attribute of an EEG signal, such as frequency, and the delivery of at least one of the pulses is based at least partly on the measured or real-time EEG signal corresponding or containing this predetermined attribute. As an example, the EEG signal may be monitored for activity within EEG frequency bands, such as Beta (14 - 30 Hz), Alpha (8 - 13 Hz), Theta (4 - 7 Hz) and Delta (1 - 3 Hz) bands, with the level of cortical arousal corresponding with the frequency of the activity, the higher the frequency the greater the arousal. In this fashion, timing of stimulation may be based on a monitored EEG signal being within a certain band of activity. As a further example, the pulse may be delivered such that it corresponds with a predicted wave or that the pulse may be delivered such that it is delivered off wave.

Some embodiments employ an excitability frequency searching algorithm to find an individual person's optimal interval or intervals, for example, the optimal interval between pulses of a pulse pair or interval between pulse pairs within a burst. Such search algorithms may consider the individual's response to stimulation in response to a currently employed interval in order to determine the optimal interval. Certain search algorithms vary a number of pulse pairs within bursts to determine an optimal number of pulse pairs within a burst. In other words, such searching algorithms determine how person 170 responds to timings of a session when these timings are varied. This enables adapting timing interval parameters of a TMS session to the brain on person 170.

Some embodiments provide for an overall treatment protocol carried out multiple times in one day, for example, from 1 to 15 times. Such treatment protocols may comprise carrying out a session of TMS stimulation 1 to 15 times a day from 2 to 7 days a week, for example on 2 to 7 consecutive days. Within some treatment protocols, the time between sessions is from 20 minutes to 5 hours, sometimes depending on the number of sessions desired during a single day.

Some embodiments comprise a step of targeting the selected region within the brain. Such region may correspond to atlas coordinates or Talairach coordinates, for example. The selected region may correspond to a functional region, such as a personalized functional region. Such functional regions may be derived from magnetic resonance imaging, MRI, functional magnetic resonance imaging, fMRI, resting state fMRI, rsfMRI or other imaging modalities. Certain embodiments rely on EEG-defined locations. Such target locations, or therapy targets, may be predefined positions within the brain, for example, dorsolateral prefrontal cortex, DLPFC, or Atlas based therapy targets. At least some methods target multiple regions, either during a single session or throughout a treatment protocol as discussed above. Some therapy protocols targeting multiple regions will target a single location on one day and then another on another day, for example, by targeting a randomly selected region from among a predefined list of target regions for each session or each day of a protocol. Certain target region or therapy targets may be anatomically defined, for example, the Broadman Area BA46 is selected to therapeutically treat certain conditions, such as depression. At least some therapy targets are selected based on a connection to depression. Some targets are selected based on an EEG response, such as a defined area that elicited direct EEG response, spectral change, EEG phase change, or EEG entropy change during a previous stimulation, such as during an initial planning stage of TMS or during TMS.

As a further example of targeting selected regions according to certain embodiments, the target region may be selected based at least in part on the target region's connectivity to the Broadman Area BA46. As another example, targeting may be accomplished by first selecting a region within the dorsolateral prefrontal cortex (DLPFC), for example BA46, such a region may be selected based on connectivity between the region within the DLPFC and other regions of the brain, for example regions which are affected in depression. Another region which may be target, for example to treat depression, is the subgenual anterior cingulate cortex (subgenual ACC). The subgenual ACC may be targeted via the DLPFC by selecting a region within the DLPFC with connectivity to the subgenual ACC. A target within the subgenual ACC may be determined, for example, through the use of fMRI, rsfMRI or other method.

Navigated transcranial magnetic stimulation, nTMS, can be applied to locate cortical muscle representations. Single TMS pulses, or pairs, bunches or pulse trains may be targeted to the motor cortex with the help of neuronavigation, for example. Measuring of motor evoked potential, MEP, amplitudes from the peripheral muscles may be carried out. The efficacy of TMS to induce MEPs has been shown to increase by when using facilitatory paired-pulse TMS, ppTMS, instead of single-pulses. Biphasic single-pulse TMS and ppTMS with inter-stimulus intervals, ISIs, of 1.4 and 2.8 ms, for example, may be applied to measure resting motor thresholds, rMTs, as a percentage of the maximal stimulator output and to determine the cortical representation areas of the right first dorsal interosseous muscle. The areas, shapes, hotspots, and center of gravities, CoGs, of the representations may be calculated. Biphasic ppTMS has potential in the mapping of cortical hand representations as an alternative for single-pulses, but with lower stimulation intensity by utilizing a cortical facilitatory mechanism. This could improve application of nTMS in subjects with low motor tract excitability.

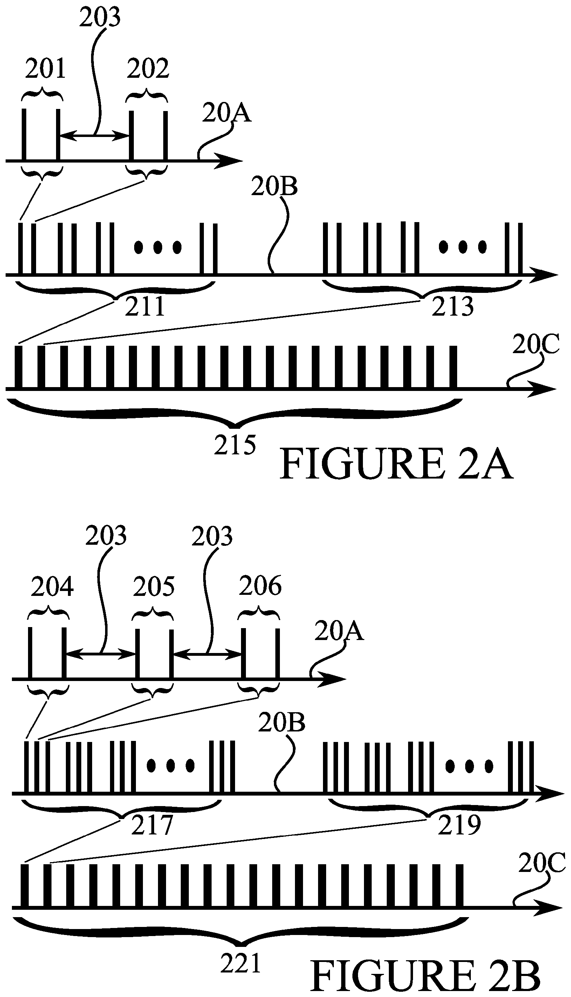

FIGURE 2A illustrates an example pulse sequence in accordance with at least some embodiments of the present invention. FIGURE 2A has three time axes 20A, 20B and 20C. Time advances from the left toward the right. On axis 20A is illustrated one burst which comprises pulse pairs 201 and 202, separated from each other by inter-pair interval 203. In this example, inter-pair interval 203 is 20 ms. The ISI between pulses of one pair 201, 203 is in this example between 1,4 ... 1,7 ms. Each burst thus comprises four individual pulses.

On axis 20B are illustrated two pulse trains 211 and 213, in this example comprising ten bursts, totalling 40 individual pulses per pulse train. The inter-burst interval in this example is 180 ms. Finally, on time axis 20C is illustrated a session 215 of twenty pulse trains 211, 213. Each vertical bar on axis 20B thus corresponds to two individual pulses. The session may be delivered with an inter-pulse train interval of eight seconds, for example, the session comprising 20 × 40 = 800 individual pulses. Each vertical bar on axis 20C thus corresponds to one pulse train 211, 213.

FIGURE 2B illustrates an example pulse sequence in accordance with at least some embodiments of the present invention. Like numbering denotes like structure as in FIGURE 2A. Here a single burst, on axis 20A, comprises three two-pulse pairs 204, 205, 206. The ISI between individual pulses of a pulse pair is 1,4 ... 1,7 ms as in FIGURE 2A. An inter-pulse interval 203 is 20 ms, as in FIGURE 2A. Pulse trains 217, 219 on axis 20B comprises ten bursts each, with an inter-burst interval of 160 ms. Each vertical bar on axis 20B thus corresponds to two individual pulses. A pulse train in FIGURE 2B has 2 × 3 × 10 = 60 individual pulses. Each vertical bar on axis 20C thus corresponds to one pulse train 217, 219.

The overall session, on axis 20C, comprises twenty of the pulse trains 217, 219 at inter-pulse train intervals of eight seconds, in this example. The session of FIGURE 2B comprises 2 × 3 × 10 × 20 = 1200 individual pulses.

A variant of the example illustrated in FIGURE 2A has a session which differs from the example described above in connection with FIGURE 2A in that the pulse trains have 20 bursts each, with the 180 ms interval, and in that the session has thirty trains, with the eight-second interval. This session has a total of 2 × 2 × 20 × 30 = 2400 individual pulses.

A variant of the example illustrated in FIGURE 2B has a session which differs from the example described above in connection with FIGURE 2B in that the session has sixty trains, with the eight-second interval. This session has a total of 2 × 3 × 10 × 60 = 3600 individual pulses.

The examples disclosed herein in connection with FIGUREs 2A and 2B, and their variants described above, may be combined with e.g. the feature that the pulses in a pulse pair are not of the same amplitude and/or with the variation in inter-burst interval and/or with the variation of the inter-pair interval.

In general, providing a session in the described form of pulse trains comprising bursts, which in turn comprise pulse pairs, enhances the benefits of TMS, since human neurons adapt to stimulation patterns that are too monotonous, which decreases the effect of TMS. Thus rendering the pulse intervals in a session more complex in form enables TMS sessions to more effectively couple with the brain of person 170.

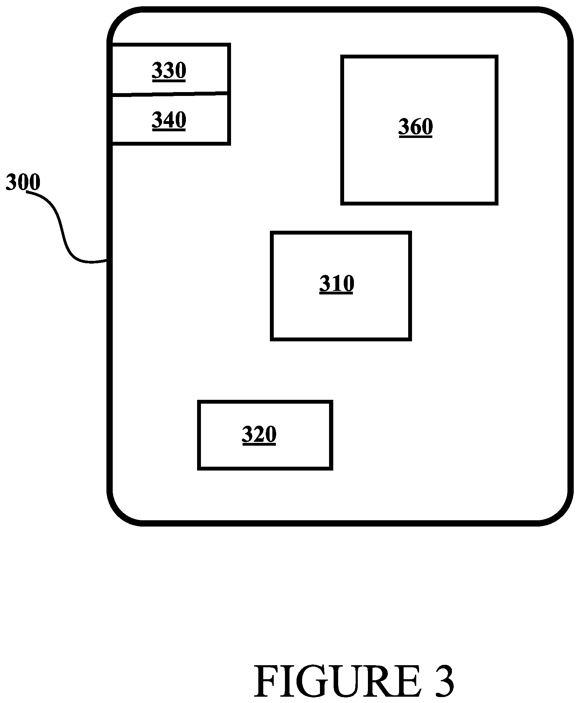

FIGURE 3 illustrates an example apparatus capable of supporting at least some embodiments of the present invention. Illustrated is device 300, which may comprise, for example, a controller 150 of FIGURE 1. Comprised in device 300 is processor 310, which may comprise, for example, a single- or multi-core processor wherein a single-core processor comprises one processing core and a multi-core processor comprises more than one processing core. Processor 310 may comprise, in general, a control device. Processor 310 may comprise more than one processor. Processor 310 may be a control device. A processing core may comprise, for example, a Cortex-A8 processing core manufactured by ARM Holdings or a Zen processing core designed by Advanced Micro Devices Corporation. Processor 310 may comprise at least one Qualcomm Snapdragon and/or Intel Core processor. Processor 310 may comprise at least one application-specific integrated circuit, ASIC. Processor 310 may comprise at least one field-programmable gate array, FPGA. Processor 310 may be means for performing method steps in device 300. Processor 310 may be configured, at least in part by computer instructions, to perform actions.

A processor may comprise circuitry, or be constituted as circuitry or circuitries, the circuitry or circuitries being configured to perform phases of methods in accordance with embodiments described herein. For example, as used in this application, the term circuitry covers an implementation of merely a hardware circuit or processor (or multiple processors or processing cores) or a portion of a hardware circuit or processor and its (or their) accompanying software and/or firmware. The term circuitry also covers, for example and if applicable to the particular claim element, a baseband integrated circuit or processor integrated circuit for a mobile device or a similar integrated circuit in server, a cellular network device, or other computing or network device.

Device 300 may comprise memory 320. Memory 320 may comprise random-access memory and/or permanent memory. Memory 320 may comprise at least one RAM chip. Memory 320 may comprise solid-state, magnetic, optical and/or holographic memory, for example. Memory 320 may be at least in part accessible to processor 310. Memory 320 may be at least in part comprised in processor 310. Memory 320 may be means for storing information. Memory 320 may comprise computer instructions that processor 310 is configured to execute. When computer instructions configured to cause processor 310 to perform certain actions are stored in memory 320, and device 300 overall is configured to run under the direction of processor 310 using computer instructions from memory 320, processor 310 and/or its at least one processing core may be considered to be configured to perform said certain actions. Memory 320 may be at least in part comprised in processor 310. Memory 320 may be at least in part external to device 300 but accessible to device 300.

Device 300 may comprise a transmitter 330. Device 300 may comprise a receiver 340. Transmitter 330 and receiver 340 may be configured to transmit and receive, respectively, information in accordance with at least one cellular or non-cellular standard. Transmitter 330 may comprise more than one transmitter. Receiver 340 may comprise more than one receiver. Transmitter 330 and/or receiver 340 may be configured to operate in accordance with global system for mobile communication, GSM, wideband code division multiple access, WCDMA, 5G, long term evolution, LTE, IS-95, wireless local area network, WLAN, Ethernet and/or worldwide interoperability for microwave access, WiMAX, standards, for example.

Device 300 may comprise user interface, UI, 360. UI 360 may comprise at least one of a display, a keyboard, a touchscreen, a vibrator arranged to signal to a user by causing device 300 to vibrate, a speaker and a microphone. A user may be able to operate device 300 via UI 360, for example to configure TMS session parameters, such as timing intervals and pulse train lengths.

Processor 310 may be furnished with a transmitter arranged to output information from processor 310, via electrical leads internal to device 300, to other devices comprised in device 300. Such a transmitter may comprise a serial bus transmitter arranged to, for example, output information via at least one electrical lead to memory 320 for storage therein. Alternatively to a serial bus, the transmitter may comprise a parallel bus transmitter. Likewise processor 310 may comprise a receiver arranged to receive information in processor 310, via electrical leads internal to device 300, from other devices comprised in device 300. Such a receiver may comprise a serial bus receiver arranged to, for example, receive information via at least one electrical lead from receiver 340 for processing in processor 310. Alternatively to a serial bus, the receiver may comprise a parallel bus receiver.

Device 300 may comprise further devices not illustrated in FIGURE 3. For example, device 300 may comprise an interface to pedal 155, TMS stimulation device 110 and/or tracking system 130. In some embodiments, device 300 comprises TMS stimulation device 110.

Processor 310, memory 320, transmitter 330, receiver 340 and/or UI 360 may be interconnected by electrical leads internal to device 300 in a multitude of different ways. For example, each of the aforementioned devices may be separately connected to a master bus internal to device 300, to allow for the devices to exchange information. However, as the skilled person will appreciate, this is only one example and depending on the embodiment various ways of interconnecting at least two of the aforementioned devices may be selected without departing from the scope of the present invention.

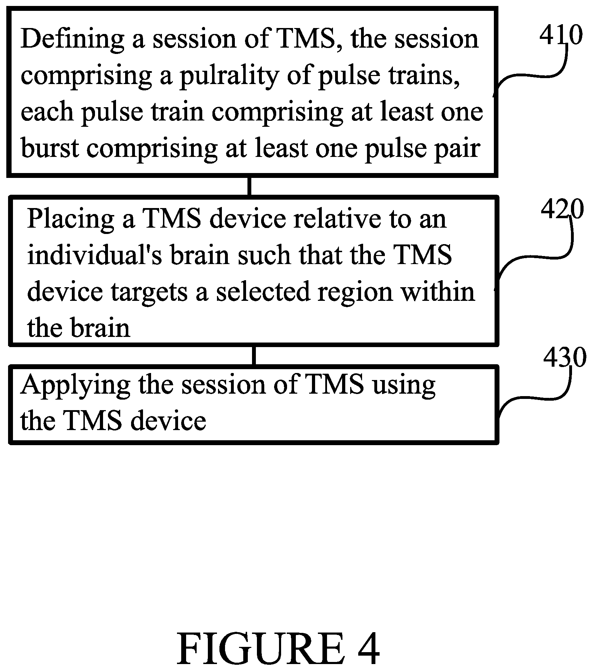

FIGURE 4 is a flow chart illustrating a sequence of events in accordance with at least some embodiments of the present invention.

Phase 410 comprises defining a session of transcranial magnetic stimulation, TMS, the session of TMS comprising a plurality of pulse trains, each pulse train comprising at least one burst and each burst comprising at least one pulse pair. Phase 420 comprises placing a TMS device relative to an individual's brain such that the TMS device targets a selected region within the brain. Phase 430 comprises applying the session of TMS using the TMS device. Herein, each pulse train comprises 2 to 20 bursts, the interval between bursts being 80 to 500 ms, the interval between pulses of the at least one pulse pair is 1 to 20 ms, and the interval between pulse trains is 1 to 60 seconds, for example less than 30 seconds, or 4 to 30 seconds. The applying may comprise, for example, controller 150 sending signals to TMS stimulation device 110 to control it to deliver the stimulation pulses.

In at least some embodiments the TMS device is held by a user. For example, the TMS device may be supported by an arm or static coil holder and manipulated into a desired location by a user. The static coil holder may be non-robotic or lack any motorized components.

It is to be understood that the embodiments of the invention disclosed are not limited to the particular structures, process steps, or materials disclosed herein, but are extended to equivalents thereof as would be recognized by those ordinarily skilled in the relevant arts. It should also be understood that terminology employed herein is used for the purpose of describing particular embodiments only and is not intended to be limiting.

Reference throughout this specification to one embodiment or an embodiment means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment. Where reference is made to a numerical value using a term such as, for example, about or substantially, the exact numerical value is also disclosed.

As used herein, a plurality of items, structural elements, compositional elements, and/or materials may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary. In addition, various embodiments and example of the present invention may be referred to herein along with alternatives for the various components thereof. It is understood that such embodiments, examples, and alternatives are not to be construed as de facto equivalents of one another, but are to be considered as separate and autonomous representations of the present invention.

Furthermore, the described features, structures, or characteristics may be combined in any suitable manner in one or more embodiments. In the preceding description, numerous specific details are provided, such as examples of lengths, widths, shapes, etc., to provide a thorough understanding of embodiments of the invention. One skilled in the relevant art will recognize, however, that the invention can be practiced without one or more of the specific details, or with other methods, components, materials, etc. In other instances, well-known structures, materials, or operations are not shown or described in detail to avoid obscuring aspects of the invention.

The verbs "to comprise" and "to include" are used in this document as open limitations that neither exclude nor require the existence of also un-recited features. The features recited in depending claims are mutually freely combinable unless otherwise explicitly stated. Furthermore, it is to be understood that the use of "a" or "an", that is, a singular form, throughout this document does not exclude a plurality.

## Claims

1. An apparatus (300) for the delivery of a session of therapeutic transcranial magnetic stimulation, TMS, the apparatus (300) comprising at least one processing core (310), at least one memory (320) including computer program code, the at least one memory and the computer program code being configured to, with the at least one processing core (310), cause the apparatus (300) at least to:
- define a session (215) of transcranial magnetic stimulation, TMS, the session (215) of TMS comprising a plurality of pulse trains (211, 213), the interval between pulse trains (211, 213) being 1 to 60 seconds, each pulse train (211, 213) comprising at least two bursts and each burst comprising at least one pulse pair or pulse triplet or pulse quadruplet, the interval (203) between pulses of the at least one pulse pair or pulse triplet or pulse quadruplet (201, 202, 204, 205, 206) is 1 to 5 ms, and
- send signals configured to cause delivery of the session of TMS,
**characterized in that**
- each burst comprises a plurality of the pulse pairs or pulse triplets or pulse quadruplets (201, 202, 204, 205, 206), such as 2 to 3 pulse pairs or pulse triplets or pulse quadruplets (201, 202, 204, 205, 206), and the interval between the pulse pairs or pulse triplets or pulse quadruplets (201, 202, 204, 205, 206) is 10 to 30 ms, preferably 20 ms.

2. The apparatus (300) of claim 1, wherein there are at least 8 pulse trains (211, 213), preferably 10 to 40 pulse trains (211, 213), more preferably 20 pulse trains (211, 213) delivered within 3 to 15 minutes, preferably within 8 minutes.

3. The apparatus (300) of any preceding claim, wherein the interval (203) between the pulses of the pulse pairs or pulse triplets or pulse quadruplets (201, 202, 204, 205, 206) is 1 to 2,8 ms, preferably 1,2 to 1,7 ms.

4. The apparatus (300) of any preceding claim, wherein the interval between bursts is 100 to 300 ms, preferably 140 to 200 ms, more preferably 150 to 160 ms.

5. The apparatus (300) according to claim 1, wherein the interval between pulse pairs or pulse triplets or pulse quadruplets (201, 202, 204, 205, 206) varies within the session (215), for example, within an individual burst or between bursts.

6. The apparatus (300) of any preceding claim, wherein the pulse pairs (201, 202, 204, 205, 206) are bi-phasic pulse pairs.

7. The apparatus (300) of any preceding claim, wherein the interval between pulses of the pulse pairs or pulse triplets or pulse quadruplets (201, 202, 204, 205, 206) varies within the session.

8. The apparatus (300) of any preceding claim, wherein the interval between pulse trains (211, 213) varies within the session (215).

9. The apparatus (300) of any preceding claim, wherein the number of bursts within a pulse train (211, 213) varies within the session (215).

10. The apparatus (300) of any preceding claim, wherein the second pulse in each pulse pair or pulse triplet or pulse quadruplet (201, 202, 204, 205, 206) is from 50% to 180% of the amplitude of the first pulse.

11. The apparatus (300) of any preceding claim, wherein the first pulse and second pulse of each pulse pair or pulse triplet or pulse quadruplet (201, 202, 204, 205, 206) vary in amplitude relative to each other.

12. The apparatus (300) of any preceding claim, the interval (203) between pulses of one of the pulse pairs or pulse triplets or pulse quadruplets (201, 202, 204, 205, 206) is 1,4 ms and the interval between pulses of an other pulse pair or pulse triplet or pulse quadruplet is 1,6 ms.

13. The apparatus (300) of any preceding claim, wherein the apparatus is further configured to receive real-time representations of brain activity, such as EEG readings, wherein at least one of: the initial pulse in at least one pulse train (211, 213) and at least some intervals, are selected based at least partly on the real-time representations of brain activity.

## Patentansprüche

1. Einrichtung (300) zur Abgabe einer Sitzung therapeutischer transkranieller Magnetstimulation, TMS, wobei die Einrichtung (300) mindestens einen Prozessorkern (310), mindestens einen Speicher (320), der Computerprogrammcode beinhaltet, umfasst, wobei der mindestens eine Speicher und der Computerprogrammcode so konfiguriert sind, dass sie mit dem mindestens einen Prozessorkern (310) die Einrichtung (300) veranlassen, um mindestens:
- eine Sitzung (215) transkranieller Magnetstimulation, TMS, zu definieren, wobei die TMS-Sitzung (215) eine Vielzahl von Impulsfolgen (211, 213) umfasst, wobei das Intervall zwischen Impulsfolgen (211, 213) 1 bis 60 Sekunden beträgt, wobei jede Impulsfolge (211, 213) mindestens zwei Bursts umfasst und jeder Burst mindestens ein Impulspaar oder Impulstripel oder Impulsquadrupel umfasst, wobei das Intervall (203) zwischen Impulsen des mindestens einen Impulspaares oder Impulstripels oder Impulsquadrupels (201, 202, 204, 205, 206) 1 bis 5 ms beträgt, und
- Signale zu senden, die so konfiguriert sind, dass sie Abgabe der TMS-Sitzung veranlassen,
**dadurch gekennzeichnet, dass**
- jeder Burst eine Vielzahl der Impulspaare oder Impulstripel oder Impulsquadrupel (201, 202, 204, 205, 206), wie etwa 2 bis 3 Impulspaare oder Impulstripel oder Impulsquadrupel (201, 202, 204, 205, 206) umfasst,
und das Intervall zwischen den Impulspaaren oder Impulstripeln oder Impulsquadrupeln (201, 202, 204, 205, 206) 10 bis 30 ms, bevorzugt 20 ms, beträgt.

2. Einrichtung (300) nach Anspruch 1, wobei es mindestens 8 Impulsfolgen (211, 213), bevorzugt 10 bis 40 Impulsfolgen (211, 213), bevorzugter 20 Impulsfolgen (211, 213) gibt, die innerhalb von 3 bis 15 Minuten, bevorzugt innerhalb von 8 Minuten, abgegeben werden.

3. Einrichtung (300) nach einem vorstehenden Anspruch, wobei das Intervall (203) zwischen den Impulsen der Impulspaare oder Impulstripel oder Impulsquadrupel (201, 202, 204, 205, 206) 1 bis 2,8 ms, bevorzugt 1,2 bis 1,7 ms, beträgt.

4. Einrichtung (300) nach einem vorstehenden Anspruch, wobei das Intervall zwischen Bursts 100 bis 300 ms, bevorzugt 140 bis 200 ms, bevorzugter 150 bis 160 ms, beträgt.

5. Einrichtung (300) nach Anspruch 1, wobei das Intervall zwischen Impulspaaren oder Impulstripeln oder Impulsquadrupeln (201, 202, 204, 205, 206) innerhalb der Sitzung (215) variiert, zum Beispiel innerhalb eines einzelnen Bursts oder zwischen Bursts.

6. Einrichtung (300) nach einem vorstehenden Anspruch, wobei die Impulspaare (201, 202, 204, 205, 206) biphasische Impulspaare sind.

7. Einrichtung (300) nach einem vorstehenden Anspruch, wobei das Intervall zwischen Impulsen der Impulspaare oder Impulstripel oder Impulsquadrupel (201, 202, 204, 205, 206) innerhalb der Sitzung variiert.

8. Einrichtung (300) nach einem vorstehenden Anspruch, wobei das Intervall zwischen Impulsfolgen (211, 213) innerhalb der Sitzung (215) variiert.

9. Einrichtung (300) nach einem vorstehenden Anspruch, wobei die Anzahl von Bursts innerhalb einer Impulsfolge (211, 213) innerhalb der Sitzung (215) variiert.

10. Einrichtung (300) nach einem vorstehenden Anspruch, wobei der zweite Impuls in jedem Impulspaar oder Impulstripel oder Impulsquadrupel (201, 202, 204, 205, 206) 50 % bis 180 % der Amplitude des ersten Impulses beträgt.

11. Einrichtung (300) nach einem vorstehenden Anspruch, wobei der erste Impuls und der zweite Impuls jedes Impulspaares oder Impulstripels oder Impulsquadrupels (201, 202, 204, 205, 206) amplitudenmäßig zueinander variieren.

12. Einrichtung (300) nach einem vorstehenden Anspruch, wobei das Intervall (203) zwischen Impulsen eines der Impulspaare oder Impulstripel oder Impulsquadrupel (201, 202, 204, 205, 206) 1,4 ms beträgt und das Intervall zwischen Impulsen eines anderen Impulspaares oder Impulstripels oder Impulsquadrupels 1,6 ms beträgt.

13. Einrichtung (300) nach einem vorstehenden Anspruch, wobei die Einrichtung weiter so konfiguriert ist, dass sie Echtzeitdarstellungen der Gehirnaktivität, wie etwa EEG-Messwerte, empfängt, wobei mindestens eines von: dem ersten Impuls in mindestens einer Impulsfolge (211, 213) und mindestens einigen Intervallen, mindestens teilweise auf Basis der Echtzeitdarstellungen der Gehirnaktivität ausgewählt werden.

## Revendications

1. Appareil (300) pour l'administration d'une session de stimulation magnétique transcrânienne, TMS, thérapeutique, l'appareil (300) comprenant au moins un cœur de traitement (310), au moins une mémoire (320) incluant un code de programme informatique, la au moins une mémoire et le code de programme informatique étant configurés pour, avec le au moins un cœur de traitement (310), amener l'appareil (300) à au moins :
- définir une session (215) de stimulation magnétique transcrânienne, TMS, la session (215) de TMS comprenant une pluralité de trains d'impulsions (211, 213), l'intervalle entre les trains d'impulsions (211, 213) étant de 1 à 60 secondes, chaque train d'impulsions (211, 213) comprenant au moins deux salves et chaque salve comprenant au moins une paire d'impulsions ou au moins un triplet d'impulsions ou au moins un quadruplet d'impulsions, l'intervalle (203) entre les impulsions des au moins une paire d'impulsions ou au moins un triplet d'impulsions ou au moins un quadruplet d'impulsions (201, 202, 204, 205, 206) est de 1 à 5 ms, et
- envoyer des signaux configurés pour provoquer l'administration de la session de TMS,
**caractérisé en ce que**
- chaque salve comprend une pluralité des paires d'impulsions ou triplets d'impulsions ou quadruplets d'impulsions (201, 202, 204, 205, 206), par exemple 2 à 3 paires d'impulsions ou triplets d'impulsions ou quadruplets d'impulsions (201, 202, 204, 205, 206),
et l'intervalle entre les paires d'impulsions ou triplets d'impulsions ou quadruplets d'impulsions (201, 202, 204, 205, 206) est de 10 à 30 ms, préférentiellement de 20 ms.

2. Appareil (300) selon la revendication 1, dans lequel il y a au moins 8 trains d'impulsions (211, 213), préférentiellement 10 à 40 trains d'impulsions (211, 213), plus préférentiellement 20 trains d'impulsions (211, 213) délivrés en 3 à 15 minutes, préférentiellement en 8 minutes.

3. Appareil (300) selon une quelconque revendication précédente, dans lequel l'intervalle (203) entre les impulsions des paires d'impulsions ou triplets d'impulsions ou quadruplets d'impulsions (201, 202, 204, 205, 206) est de 1 à 2,8 ms, préférentiellement de 1,2 à 1,7 ms.

4. Appareil (300) selon une quelconque revendication précédente, dans lequel l'intervalle entre les salves est de 100 à 300 ms, préférentiellement de 140 à 200 ms, plus préférentiellement de 150 à 160 ms.

5. Appareil (300) selon la revendication 1, dans lequel l'intervalle entre les paires d'impulsions ou triplets d'impulsions ou quadruplets d'impulsions (201, 202, 204, 205, 206) varie au cours de la session (215), par exemple, au cours d'une salve individuelle ou entre des salves.

6. Appareil (300) selon une quelconque revendication précédente, dans lequel les paires d'impulsions (201, 202, 204, 205, 206) sont des paires d'impulsions biphasiques.

7. Appareil (300) selon une quelconque revendication précédente, dans lequel l'intervalle entre les impulsions des paires d'impulsions ou triplets d'impulsions ou quadruplets d'impulsions (201, 202, 204, 205, 206) varie au cours de la session.

8. Appareil (300) selon une quelconque revendication précédente, dans lequel l'intervalle entre les trains d'impulsions (211, 213) varie au cours de la session (215).

9. Appareil (300) selon une quelconque revendication précédente, dans lequel le nombre de salves dans un train d'impulsions (211, 213) varie au cours de la session (215).

10. Appareil (300) selon une quelconque revendication précédente, dans lequel la deuxième impulsion dans chaque paire d'impulsions ou triplet d'impulsions ou quadruplet d'impulsions (201, 202, 204, 205, 206) est de 50 % à 180 % de l'amplitude de la première impulsion.

11. Appareil (300) selon une quelconque revendication précédente, dans lequel la première impulsion et la deuxième impulsion de chaque paire d'impulsions ou triplet d'impulsions ou quadruplet d'impulsions (201, 202, 204, 205, 206) varient en amplitude l'une par rapport à l'autre.

12. Appareil (300) selon une quelconque revendication précédente, l'intervalle (203) entre les impulsions d'un parmi les paires d'impulsions ou triplets d'impulsions ou quadruplets d'impulsions (201, 202, 204, 205, 206) est de 1,4 ms et l'intervalle entre les impulsions d'une autre paire d'impulsions ou d'un autre triplet d'impulsions ou d'un autre quadruplet d'impulsions est de 1,6 ms.

13. Appareil (300) selon une quelconque revendication précédente, dans lequel l'appareil est en outre configuré pour recevoir des représentations en temps réel de l'activité cérébrale, telles que des lectures d'EEG, dans lequel au moins un parmi : l'impulsion initiale dans au moins un train d'impulsions (211, 213) et au moins certains intervalles, est sélectionné au moins en partie sur la base des représentations en temps réel de l'activité cérébrale.
